# EUROPEAN PATENT SPECIFICATION

(11) **EP 4 197 833 B1**
(45) Date of publication and mention of the grant of the patent: **04.09.2024**
(21) Application number: 21215574.1
(22) Date of filing: 17.12.2021
(51) Int. Cl.: B60H 1/26, B60H 3/00, B60H 1/00, B60J 7/16, A61L 9/20

(54) **A VEHICLE HATCH ASSEMBLY**
LUKENANORDNUNG FÜR FAHRZEUGE
ENSEMBLE HAYON DE VÉHICULE

(43) Date of publication of application: 21.06.2023
(73) Proprietor: Valeo Thermal Commercial Vehicles Germany GmbH, 82205 Gilching (DE)
(72) Inventor: SCHOULTZ, Jan Erik, 20360 Turku (FI)
(74) Representative: Strasser, Wolfgang

(56) References cited:
- EP-A1- 3 943 117
- JP-A- 2002 178 745
- US-B1- 6 336 678

## Description

### FIELD OF THE INVENTION

The invention relates to a vehicle hatch assembly, in particular to a vehicle hatch assembly adapted for air purification.

### BACKGROUND OF THE INVENTION

There is a constant need for conditioning of the air used for breathing in closed spaces. This relates to stationary spaces, like housing or office rooms, as well as to passenger transportation means, such as road vehicles, airplanes, ships etc. In this context, in addition to humidity and temperature, air quality is also an important parameter for comfort and safety of users. The air circulating within a closed space degrades over time as it is breathed in and out by users. Presence of viruses, bacteria, fungi has a negative influence on health of the passengers.

It has been known to utilize UV light sources for purification of air thanks to their germicidal properties. UV-C light, i.e. ultraviolet radiation with wavelength between 100 and 280 nm, is particularly effective at disrupting DNA of viruses such as COVID-19, so that the virus can no longer reproduce itself. However, implementation of UV light sources usually is problematic in view of safety. The beneficial effect of destroying viruses, bacteria and fungi can easily be harmful to users if they are subject to direct exposure to UV radiation. In general, utilization of UV light sources for purification on air is problematic in terms of space, cost and reliability concerns, especially for mobile applications.

Vehicles used for transportation of multiple passengers, for example buses, have significant need for supply a fresh and clean air for breathing. Additional devices mounted within the passenger compartment are known to be applied in such vehicles, in particular for purifying air to remove any viruses. These however occupy space, which could otherwise be dedicated for passengers comfort. Additionally, they require additional mounting arrangements and intrusion into existing structure and design of the vehicle.

A vehicle hatch assembly adapted to be mounted in a vehicle are known from JP2002178745 and EP3943117.

It would be desirable to provide an air purification device, which would at least partly alleviate the above-mentioned problems.

### SUMMARY OF THE INVENTION

The object of the invention is, a vehicle hatch assembly according to claim 1, adapted to be mounted in a vehicle, wherein it comprises a housing and at least one air inlet and at least one air outlet, an air propulsion device configured to force the air movement between the air inlet and the air outlet, and a UV light source located within the housing between the air inlet and the air outlet.

Preferably, the air inlet and the air outlet are located on a first side of the vehicle hatch assembly, the vehicle hatch assembly being configured to exchange air with a second side of the vehicle hatch assembly located in an opposite way with respect to the first side.

Preferably, it comprises an opening arrangement configured as air inlet and/or outlet, comprising dedicated air propulsion means to force air movement between the first side and the second side through the vehicle hatch assembly.

Preferably, it further comprises a window section configured to at least partly transmit light between the first side and the second side.

According to the invention, it comprises at least one light trap configured to prevent the UV light from escaping the housing through the air inlet and/or the air outlet.

Preferably, the light trap has a first wall and a second wall extending in parallel to each other at a distance, wherein the first wall comprises first air openings and the second wall comprises second air openings, the first air openings being shifted with respect to the second air openings in a direction perpendicular to the extension direction of the first wall and the second wall.

Preferably, it comprises a first air propulsion device dedicated for the air inlet and a second air propulsion device dedicated for the air outlet, wherein the UV light source is located therebetween.

Preferably, it comprises a first light trap arranged between the UV light source and the first air propulsion device and a second light trap arranged between the UV light source and the second air propulsion device.

Preferably, any light trap comprises a UV protection coating.

Preferably, the coating is a black matte powder coating.

Preferably, it is an emergency exit hatch, comprising a door adapted for switching between a closed position and an opened position.

Preferably, it comprises an emergency handle adapted for opening and closing the vehicle hatch assembly.

Another object of the invention is a vehicle, in particular a bus, comprising a vehicle hatch assembly according to any of claims 1 to 11.

### BRIEF DESCRITPTION OF DRAWINGS

Examples of the invention will be apparent from and described in detail with reference to the accompanying drawings, in which:
Fig. 1 shows the vehicle hatch assembly in an isometric view from the top side;
Fig. 2 shows the vehicle hatch assembly in an isometric view from the bottom side;
Fig. 3 shows the vehicle hatch assembly in an isometric view from the top side with top cover removed;
Fig. 4 shows an enlarged portion of the view of Fig. 4;
Fig. 5 shows schematically side view of another embodiment of the invention;
Figs. 6a and 6b show schematically side view of the vehicle hatch assembly in a closed and open position.

### DETAILED DESCRIPTION OF EMBODIMENTS

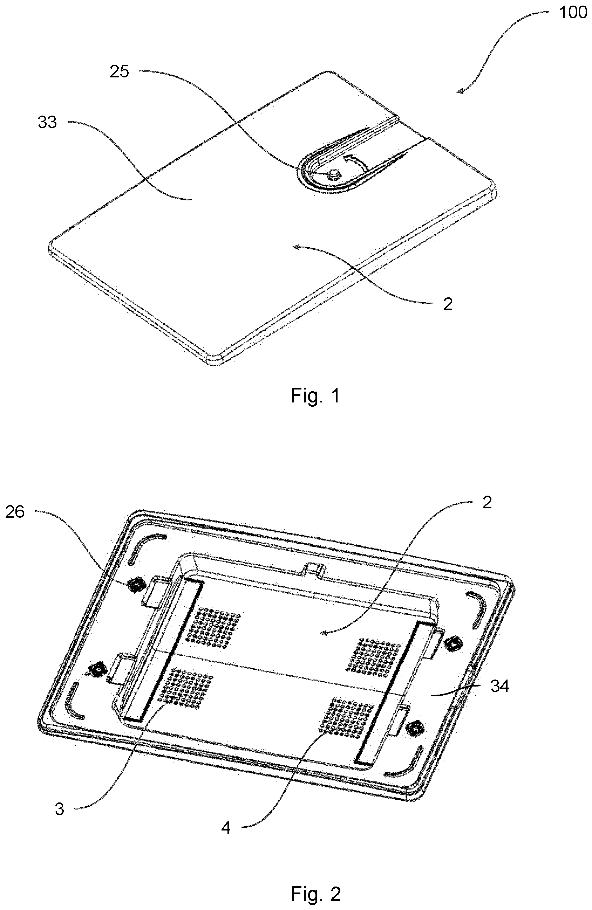

Fig. 1 shows the vehicle hatch assembly 100 in an isometric view from the top side. The vehicle hatch assembly comprises a housing 2 with a top cover 33. Optionally, it may comprise an emergency handle 25 adapted for opening and closing the assembly. The vehicle hatch assembly 100 is configured to be mounted in place of traditional hatch assembly in a roof of a vehicle, e.g. bus, in a known manner.

The Fig. 2 shows the vehicle hatch assembly 100 in an isometric view from the bottom side. The housing 2 has bottom plate 34 with at least one air inlet 3 and at least one air outlet 4. The housing 2 is shaped to define internal volume for air. Preferably, the housing 2 is air-tight between the air inlet 3 and the air outlet 4. In the shown case, the housing 2 has substantially a flattened cuboid shape. It may have closing members 26 for removable fixing of the cover 33 to the bottom plate 34.

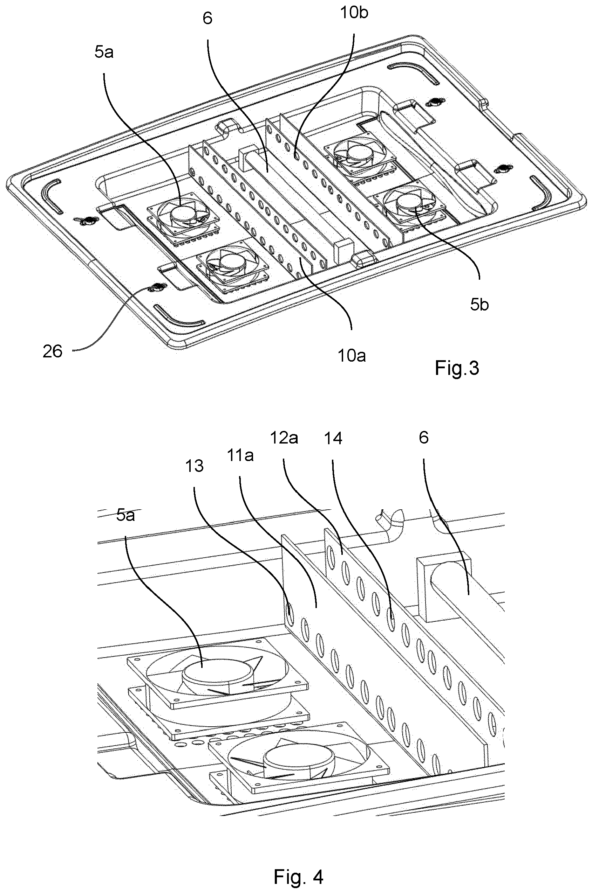

Fig. 3 shows the vehicle hatch assembly 100 in an isometric view from the top side with top cover 33 removed. The vehicle hatch assembly 100 comprises at least one air propulsion device 5a, 5b configured to force the air movement between the air inlet 3 and the air outlet 4. In this case, it may be in form of a known blower. According to needs, the number and configuration of air propulsion devices 5a, 5b may vary. In the examples shown in the figures, there are two first air propulsion devices 5a at the air inlet 3 and two second air propulsion devices 5b at the air outlet 4. The vehicle hatch assembly 100 further comprises a UV light source 6, located within the housing 2 between the air inlet 3 and the air outlet 4. Preferably, there is a first air propulsion device 5a dedicated for the air inlet 3 and a second air propulsion device 5b dedicated for the air outlet 4, wherein the UV light source 6 is located therebetween. By providing a UV light source 6, the air travelling between the air inlet 3 and the air outlet 4 is subject to UV radiation so that airborne mold bacteria can be killed, and viruses floating in the air can be inactivated. Consequently, the air leaving the vehicle hatch assembly 100 is safe for users. Preferably, the UV light source 6 produces UV-C light with 254nm wavelength. This wavelength has been proven to be optimal for neutralizing harmful viruses, for example Covid-19. The vehicle hatch assembly 100 further comprises at least one light trap 10a, 10b, preferably two, configured to prevent the UV light from escaping the housing 2 through the air inlet 3 and/or the air outlet 4. The housing 2 may comprise an electrical ballast for the UV light source 6, as well as an electronic control unit.

Preferably, any light trap 10a, 10b directly faces the UV light source 6. In this way, the size of the vehicle hatch assembly 100 can be optimized for an efficient operation with a compact packaging. In the shown example, the assembly comprises a first light trap 10a arranged between the UV light source 6 and the first air propulsion device 5a and a second light trap 10b arranged between the UV light source 6 and the second air propulsion device 5b.

Fig. 4 shows an enlarged portion of the view of Fig. 4. As it can be seen, the first light trap 10a has a first wall 11a and a second wall 12a extending in parallel to each other at a distance. The first wall 11a comprises first air openings 13 and the second wall 12a comprises second air openings 14. The first air openings 13 are shifted with respect to the second air openings 14 in a direction perpendicular to the extension direction of the first wall 11a and the second wall 12a. In this manner, the UV light is prevented from directly reaching the propulsion device 5a and the inlet 3. The second light trap 10b is configured analogously in order to protect the second propulsion device 5b and the outlet 4. Consequently, the passengers are safe from harmful direct exposure. Preferably, the light trap 10a, 10b comprises a UV protection coating. One example of such coating is a black matte powder coating.

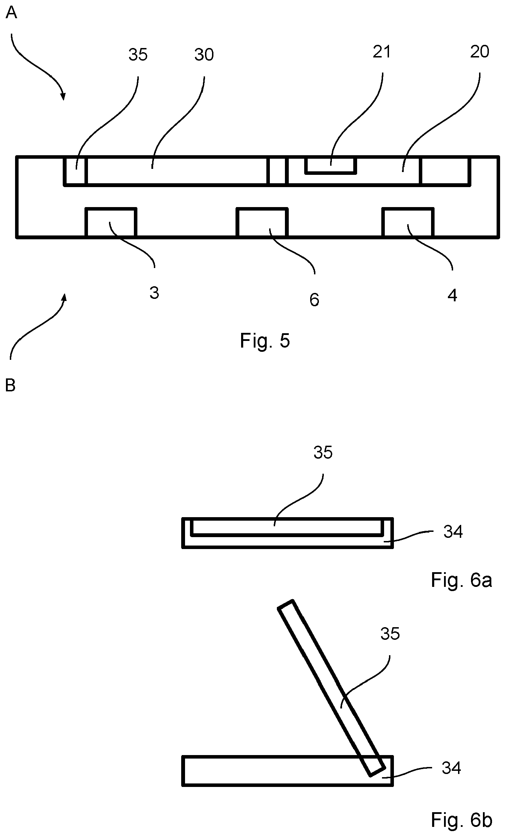

Fig. 5 shows schematically side view of another embodiment of the invention. The air inlet 3 and the air outlet 4 are located on a first side A of the vehicle hatch assembly 100. The vehicle hatch assembly 100 may be configured to exchange air with a second side B of the vehicle hatch assembly 100 located in an opposite way with respect to the first side A. It may comprise an opening arrangement 20 configured as air inlet and/or outlet, with dedicated air propulsion means 21 to force air movement between the first side A and the second side B through the vehicle hatch assembly 100. In this manner, the vehicle hatch assembly 100 may draw fresh air from the outside and/or expel air from the inside of the vehicle. It may further comprise a window section 30 configured to at least partly transmit light between the first side A and the second side B.

Figs. 6a and 6b show schematically side view of the vehicle hatch assembly in a closed and open position. The vehicle hatch assembly 100 may be an emergency exit hatch, comprising a door 35 adapted for switching between a closed position, shown in Fig. 6a, and an opened position, shown in Fig. 6b.

Because any bus has an emergency exit hatch as default, the vehicle hatch assembly 100 according to invention can be easily placed in most such existing vehicles. Because the vehicle hatch assembly 100 is allowed to have standard frame, it can be mounted in already existing roof openings without any substantial modification. The vehicle hatch assembly 100 according to invention does not take any additional space on the vehicle and additional purification units are not needed to ensure that the air circulating within the bus is purified.

The invention solves the space requirement, substantially reduces the number of components in the vehicle and saves weight. The invention can be implemented in several ways and on several bases: a mere emergency exit door, ventilation hatch, light-providing window etc.

Other variations to the disclosed embodiments can be understood and effected by those skilled in the art in practicing the claimed invention, from a study of drawings, the disclosure, and within the scope of the appended claims. The mere fact that certain measures are recited in mutually different dependent claims does not indicate that a combination of these measures cannot be used to the advantage.

## Claims

1. A vehicle hatch assembly (100) adapted to be mounted in a vehicle, and comprises a housing (2) and at least one air inlet (3) and at least one air outlet (4), an air propulsion device (5a, 5b) configured to force the air movement between the air inlet (3) and the air outlet (4), and a UV light source (6) located within the housing (2) between the air inlet (3) and the air outlet (4) **characterized in that** it further comprises at least one light trap (10a, 10b) configured to prevent the UV light from escaping the housing (2) through the air inlet (3) and/or the air outlet (4).

2. A vehicle hatch assembly (100) according to claim 1, wherein the air inlet (3) and the air outlet (4) are located on a first side (A) of the vehicle hatch assembly (100), the vehicle hatch assembly (100) being configured to exchange air with a second side (B) of the vehicle hatch assembly (100) located in an opposite way with respect to the first side (A).

3. A vehicle hatch assembly (100) according to claim 2, wherein it comprises an opening arrangement (20) configured as air inlet and/or outlet, comprising dedicated air propulsion means (21) to force air movement between the first side (A) and the second side (B) through the vehicle hatch assembly (100).

4. A vehicle hatch assembly (100) according to any preceding claim, wherein it further comprises a window section (30) configured to at least partly transmit light between the first side (A) and the second side (B).

5. A vehicle hatch assembly according to any preceding claim, wherein the light trap (10a, 10b) has a first wall (11a) and a second wall (12a) extending in parallel to each other at a distance, wherein the first wall (11a) comprises first air openings (13) and the second wall (12a) comprises second air openings (14), the first air openings (13) being shifted with respect to the second air openings (14) in a direction perpendicular to the extension direction of the first wall (11a) and the second wall (12a).

6. A vehicle hatch assembly according to any preceding claim, comprising a first air propulsion device (5a) dedicated for the air inlet (3) and a second air propulsion device (5b) dedicated for the air outlet (4), wherein the UV light source (6) is located therebetween.

7. A vehicle hatch assembly according to claim 6, comprising a first light trap (10a) arranged between the UV light source (6) and the first air propulsion device (5a) and a second light trap (10b) arranged between the UV light source (6) and the second air propulsion device (5b).

8. A vehicle hatch assembly according to any preceding claim, wherein any light trap (10a, 10b) comprises a UV protection coating.

9. A vehicle hatch assembly according to the preceding claim, wherein the coating is a black matte powder coating.

10. A vehicle hatch assembly (100) according to claim 1, wherein it is an emergency exit hatch, comprising a door (35) adapted for switching between a closed position and an opened position.

11. A vehicle hatch assembly (100) according to claim 10, wherein it comprises an emergency handle (25) adapted for opening and closing the vehicle hatch assembly (100).

12. A vehicle, in particular a bus, comprising a vehicle hatch assembly (100) according to any preceding claim.

## Patentansprüche

1. Fahrzeuglukenbaugruppe (100), die dazu geeignet ist, in einem Fahrzeug montiert zu sein, und ein Gehäuse (2) und mindestens einen Lufteinlass (3) und mindestens einen Luftauslass (4), eine Luftantriebsvorrichtung (5a, 5b), die dazu ausgelegt ist, die Luftbewegung zwischen dem Lufteinlass (3) und dem Luftauslass (4) zu erzwingen, und eine in dem Gehäuse (2) zwischen dem Lufteinlass (3) und dem Luftauslass (4) angeordnete UV-Lichtquelle (6) umfasst, **dadurch gekennzeichnet, dass** sie ferner mindestens eine Lichtfalle (10a, 10b) umfasst, die dazu ausgelegt ist, zu verhindern, dass das UV-Licht durch den Lufteinlass (3) und/oder den Luftauslass (4) aus dem Gehäuse (2) austritt.

2. Fahrzeuglukenbaugruppe (100) nach Anspruch 1, wobei der Lufteinlass (3) und der Luftauslass (4) auf einer ersten Seite (A) der Fahrzeuglukenbaugruppe (100) angeordnet sind, wobei die Fahrzeuglukenbaugruppe (100) dazu ausgelegt ist, Luft mit einer zweiten Seite (B) der Fahrzeuglukenbaugruppe (100) auszutauschen, die in Bezug auf die erste Seite (A) in entgegengesetzter Weise angeordnet ist.

3. Fahrzeuglukenbaugruppe (100) nach Anspruch 2, wobei sie eine Öffnungsanordnung (20) umfasst, die als Lufteinlass und/oder -auslass ausgelegt ist, umfassend dedizierte Luftantriebsmittel (21) zum Erzwingen einer Luftbewegung zwischen der ersten Seite (A) und der zweiten Seite (B) durch die Fahrzeuglukenbaugruppe (100).

4. Fahrzeuglukenbaugruppe (100) nach einem der vorhergehenden Ansprüche, wobei sie ferner einen Fensterabschnitt (30) umfasst, der dazu ausgelegt ist, zumindest teilweise Licht zwischen der ersten Seite (A) und der zweiten Seite (B) zu übertragen.

5. Fahrzeuglukenbaugruppe nach einem der vorhergehenden Ansprüche, wobei die Lichtfalle (10a, 10b) eine erste Wand (11a) und eine zweite Wand (12a) umfasst, die sich in einem Abstand parallel zueinander erstrecken, wobei die erste Wand (11a) erste Luftöffnungen (13) umfasst und die zweite Wand (12a) zweite Luftöffnungen (14) umfasst, wobei die ersten Luftöffnungen (13) in Bezug auf die zweiten Luftöffnungen (14) in einer Richtung senkrecht zur Erstreckungsrichtung der ersten Wand (11a) und der zweiten Wand (12a) verschoben sind.

6. Fahrzeuglukenbaugruppe nach einem der vorhergehenden Ansprüche, umfassend eine erste Luftantriebsvorrichtung (5a), die für den Lufteinlass (3) dediziert ist, und eine zweite Luftantriebsvorrichtung (5b), die für den Luftauslass (4) dediziert ist, wobei die UV-Lichtquelle (6) dazwischen angeordnet ist.

7. Fahrzeuglukenbaugruppe nach Anspruch 6, umfassend eine erste Lichtfalle (10a), die zwischen der UV-Lichtquelle (6) und der ersten Luftantriebsvorrichtung (5a) angeordnet ist, und eine zweite Lichtfalle (10b), die zwischen der UV-Lichtquelle (6) und der zweiten Luftantriebsvorrichtung (5b) angeordnet ist.

8. Fahrzeuglukenbaugruppe nach einem der vorhergehenden Ansprüche, wobei jede Lichtfalle (10a, 10b) eine UV-Schutzbeschichtung umfasst.

9. Fahrzeuglukenbaugruppe nach dem vorhergehenden Anspruch, wobei die Beschichtung eine mattschwarze Pulverbeschichtung ist.

10. Fahrzeuglukenbaugruppe (100) nach Anspruch 1, wobei sie eine Notausstiegsluke ist, umfassend eine Tür (35), die zum Wechseln zwischen einer geschlossenen Position und einer geöffneten Position geeignet ist.

11. Fahrzeuglukenbaugruppe (100) nach Anspruch 10, wobei sie einen Notgriff (25) umfasst, der zum Schließen und Öffnen der Fahrzeuglukenbaugruppe (100) geeignet ist.

12. Fahrzeug, insbesondere Bus, umfassend eine Fahrzeuglukenbaugruppe (100) nach einem der vorhergehenden Ansprüche.

## Revendications

1. Ensemble volet de véhicule (100) conçu pour être monté dans un véhicule et comprenant un boîtier (2) et au moins une entrée d'air (3) et au moins une sortie d'air (4), un dispositif de propulsion d'air (5a, 5b) conçu pour forcer le mouvement d'air entre l'entrée d'air (3) et la sortie d'air (4), et une source de lumière UV (6) située à l'intérieur du boîtier (2) entre l'entrée d'air (3) et la sortie d'air (4), **caractérisé en ce qu'**il comprend en outre au moins un piège à lumière (10a, 10b) conçu pour empêcher la lumière UV de s'échapper du boîtier (2) par l'entrée d'air (3) et/ou la sortie d'air (4).

2. Ensemble volet de véhicule (100) selon la revendication 1, l'entrée d'air (3) et la sortie d'air (4) étant situées sur un premier côté (A) de l'ensemble volet de véhicule (100), l'ensemble volet de véhicule (100) étant conçu pour échanger de l'air avec un second côté (B) de l'ensemble volet de véhicule (100) situé à l'opposé du premier côté (A).

3. Ensemble volet de véhicule (100) selon la revendication 2, comprenant un agencement d'ouverture (20) conçu comme entrée et/ou sortie d'air, comprenant un moyen de propulsion d'air (21) dédié pour forcer le mouvement d'air entre le premier côté (A) et le second côté (B) à travers l'ensemble volet de véhicule (100).

4. Ensemble volet de véhicule (100) selon l'une quelconque des revendications précédentes, comprenant en outre une section de fenêtre (30) conçue pour transmettre au moins partiellement la lumière entre le premier côté (A) et le second côté (B).

5. Ensemble volet de véhicule selon l'une quelconque des revendications précédentes, le piège à lumière (10a, 10b) ayant une première paroi (11a) et une seconde paroi (12a) s'étendant parallèlement l'une à l'autre à une certaine distance, la première paroi (11a) comprenant des premières ouvertures d'air (13) et la seconde paroi (12a) comprend des secondes ouvertures d'air (14), les premières ouvertures d'air (13) étant décalées par rapport aux secondes ouvertures d'air (14) dans une direction perpendiculaire à la direction d'extension de la première paroi (11a) et de la seconde paroi (12a).

6. Ensemble volet de véhicule selon l'une quelconque des revendications précédentes, comprenant un premier dispositif de propulsion d'air (5a) dédié à l'entrée d'air (3) et un second dispositif de propulsion d'air (5b) dédié à la sortie d'air (4), la source de lumière UV (6) étant située entre les deux.

7. Ensemble volet de véhicule selon la revendication 6, comprenant un premier piège à lumière (10a) disposé entre la source de lumière UV (6) et le premier dispositif de propulsion d'air (5a) et un second piège à lumière (10b) disposé entre la source de lumière UV (6) et le second dispositif de propulsion d'air (5b).

8. Ensemble volet de véhicule selon l'une quelconque des revendications précédentes, tout piège à lumière (10a, 10b) comprenant un revêtement de protection contre les UV.

9. Ensemble volet de véhicule selon la revendication précédente, le revêtement étant un revêtement en poudre noir mat.

10. Ensemble volet de véhicule (100) selon la revendication 1, étant un volet de sortie de secours, comprenant une porte (35) conçue pour passer d'une position fermée à une position ouverte.

11. Ensemble volet de véhicule (100) selon la revendication 10, comprenant une poignée d'urgence (25) conçue pour ouvrir et fermer l'ensemble volet de véhicule (100).

12. Véhicule, en particulier autobus, comprenant un ensemble volet de véhicule (100) selon l'une quelconque des revendications précédentes.
